# EUROPEAN PATENT SPECIFICATION

(11) **EP 2 328 542 B1**
(45) Date of publication and mention of the grant of the patent: **25.04.2018**
(21) Application number: 08863197.3
(22) Date of filing: 05.12.2008
(51) Int. Cl.: A61K 8/19, A61K 8/25, A61K 8/26, A61K 8/35, A61K 8/41, A61K 8/46, A61K 8/49, A61K 8/98, A61Q 1/02, A61Q 17/04, A61Q 19/08

(54) **SUNSCREEN COMPOSITIONS COMPRISING COLOUR PIGMENS**
SONNENSCHUTZZUSAMMENSETZUNGEN MIT FARBPIGMENTEN
COMPOSITIONS D'ÉCRAN SOLAIRE COMPRENANT DES PIGMENTS DE COULEUR

(30) Priority: 14.12.2007 EP 07123286; 06.05.2008 EP 08155725
(43) Date of publication of application: 08.06.2011
(60) Divisional application: 13198908.9
(73) Proprietor: BASF SE, 67056 Ludwigshafen am Rhein (DE)
(72) Inventor: EHLIS, Thomas, 79100 Freiburg (DE); SOHN, Myriam, F-68300 Saint-louis (FR)
(74) Representative: BASF IP Association
(86) International application number: PCT/EP2008/066870
(87) International publication number: WO 2009/077356

(56) References cited:
- EP-A1- 1 129 695
- EP-A1- 1 352 639
- EP-A1- 1 709 954
- EP-A1- 1 782 792
- EP-A2- 1 792 951
- EP-A2- 1 870 078
- WO-A1-03/013468
- WO-A1-03/075875
- WO-A1-2004/098546
- WO-A1-2006/034991
- WO-A2-2004/085412
- WO-A2-2007/014848
- WO-A2-2007/071584
- DE-A1- 10 035 512
- DE-A1-102005 059 739
- DE-A1-102005 059 741
- DE-A1-102005 059 742
- DE-A1-102006 006 413
- US-A1- 2004 185 015
- US-A1- 2007 071 700
- Ciba Specialty Chemicals: "Ciba Tinosorb M - Technical Data Sheet", , 26 May 2004 (2004-05-26), pages 1-2, XP002591733, Retrieved from the Internet: URL:http://www.imminst.org/forum/index.php ?s=8325ff68f2c386b48dc4877968780287&app=co re&module=attach&section=attach&attach_rel _module=post&attach_id=7932 [retrieved on 2010-07-12]
- "Use of amino hydroxy benzophenone derivatives in sunscreen preparations", IP.COM JOURNAL, IP.COM INC., WEST HENRIETTA, NY, US, 24 August 2006 (2006-08-24), XP013115567, ISSN: 1533-0001

## Description

The present invention relates to cosmetic compositions comprising micronized and/or soluble UV absorbers and one or more color pigments.

It has long been known that prolonged exposure to that UV radiation which reaches the surface of the earth can lead to the formation of erythemas or light dermatoses, as well as to an increased incidence of skin cancers or accelerated skin aging.

Various sunscreen formulations have been proposed which include actives which are intended to counteract UV radiation, thereby inhibiting the said undesired effects on the skin.

A great number of compounds has been proposed for the use as UV protectants in sunscreen formulations, especially soluble organic UV absorbers and also insoluble micronized organic and inorganic compounds, wich become more and more common in sunscreen formulations.

Micronised, insoluble organic UV absorbers, when used in sunscreen formulations, provide excellent UV protection and have a high SPF rating. Moreover, micronised, insoluble organic UV absorbers show no tendency, under the influence of light, to generate radicals which could damage or sensitise human skin.

Unfortunately, due to light scattering sun screen compostitions comprising organic and/or inorganic particulate filters show an articulate whitening aspect when applied on the skin.

The whitening aspect increases with higher concentration of the corresponding UV filters and will therefore limit their required quantity.

Furthermore, soluble and particulate UV filters which have absorption maximum in the UV-A region are mostly not colourless since these substances will absorb also light in the visible range of the spectrum. Due to the particular colour of these UV-A filters the resulting cosmetic sun screen formulations will be coloured. The hue of such formulations (in most cases yellowish) is considered as unnatural since it derivates significantly from the natural skin hue.

Furthermore, it is known that some UV filters show significant fluorescence. Artificial illuminations with a high UV ratio therefore generate very unnatural skin tones.

WO2004/098546 discloses decorative cosmetic compositions comprising one coloring agent and at least one organic masking agent. The said compositions are tought to provide effective means for producing natural, textured tone effects (see D1, page 8, lines 1-3). The reference discloses compositions comprising benzotriazoles and talc coated with iron oxide as coloring agent (see e.g. example 4).

Surprisingly it has been found that the combination of particulate or soluble organic UV filters with one or more colour pigments provides cosmetic sunscreen formulations which have a hue that corresponds to the natural skin tone and is therefore not considered as unnatural.

Therefore, the present invention relates to a cosmetic formulation comprising
(a) particulates having an absorption in the range of 400 to 800nm selected from
   (a₁) interference-pigments based on mica and coated with iron oxide; and
   (b₂) UV filters selected from a mixture of particulate organic UV filters selected from the compounds of formula (1) 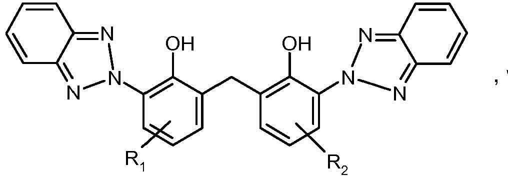 wherein
      R₁ and R₂ independently from each other are C₁-C₁₈alkyl which may be substituted by one or more radicals selected from C₁-C₄alkyl, C₅-C₁₂cycloalkyl and C₆-C₁₀aryl; and the compounds of formula 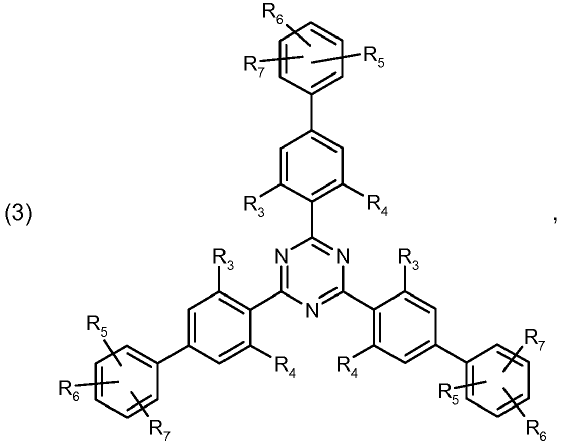 wherein
      R₃ and R₄ independently from each other are hydrogen; C₁-C₁₈alkyl; or C₆-C₁₂aryl;
      R₅, R₆ and R₇ independently from each other are hydrogen; hydroxy; halogen; C₁-C₁₈alkyl; C₁-C₁₈alkoxy; C₆-C₁₂aryl; biphenylyl; C₆-C₁₂aryloxy; C₁₋C₁₈alkylthio; carboxy; -COOM; C₁-C₁₈-alkylcarboxyl; aminocarbonyl; mono- or di-C₁-C₁₈alkylamino; C₁₋C₁₀acylamino; or -COOH; and
      M is an alkali metal ion

Of specific interest are particulates (a) listed in Table 1 below:

| Table 1c: Calisha colour pigments (Ciba): | |
|---|---|
| Treated Pearls | |
| Name | INCI Name |
| Calisha Romance Bronze Pearl OD | Mica and Red Iron Oxide and Cetyl Dimethicone |
| Calisha Romance Orange Pearl OD | Mica and Red Iron Oxide and Cetyl Dimethicone |
| Calisha Romance Red Pearl OD | Mica and Red Iron Oxide and Cetyl Dimethicone |
| Calisha Art Deco Bronze Pearl OD | Mica and Red Iron Oxide and Cetyl Dimethicone |
| Calisha Art Deco Orange Pearl OD | Mica and Red Iron Oxide and Cetyl Dimethicone |
| Calisha Art Deco Red Pearl OD | Mica and Red Iron Oxide and Cetyl Dimethicone |

The interference pigments as used in the present invention are platelet particulates. The platelet particulates preferably have a thickness of no more than about 5 micrometers, more preferably no more than about 2 micrometers, still more preferably no more than about 1 micrometer. The platelet particulates preferably have a thickness of at least about 0.02 micrometers, more preferably at least about 0.05 micrometers, even more preferably at least about 0.1 micrometers, and still more preferably at least about 0.2 micrometers.

The particle size determines the opacity and luster. The particle size is determined by measuring the diameter thickness of the particulate material. The term "diameter" as used herein, means the largest distance across the major axis of the particulate material. Diameter can be determined by any suitable method known in the art, such as particle size analyzer Mastersizer 2000 manufactured by Malvern Instruments. The interference pigments preferably have an average diameter not greater than about 200 micrometers, more preferably not greater than 150 micrometers. The interference pigments preferably have a diameter of at least about 0.1 micrometer, more preferably at least about 1.0 micrometer, even more preferably at least about 2.0 micrometer, and still more preferably at least about 5.0 micrometer.

The interference pigments comprise a multilayer structure. The center of the particulates is a flat substrate with a refractive index (RI) normally below 1.8. A wide variety of particle substrates are useful herein. Nonlimiting examples are natural mica or synthetic mica.
A layer of thin film or a multiple layer of thin films are coated on the surface of a substrate described above. The thin films are made of highly refractive materials. The refractive index of these materials is normally above 1.8.

For the multiple layer structures, the thin films can be consisted of all high refractive index materials or alternation of thin films with high and low RI materials with the high RI film as the top layer.

The interference pigment color is a function of the thickness of thin film, the thickness for a specific color may be different for different materials.

Although there are no critical limitations to the sizes of the pigment particles, preferably pigment particles having a size from about 0.01 to about 500 micrometers, more preferably having a size of about 1 to about 200 micrometers.

More preferably the particulate organic UV-filters (b₂) is Methylene Bis-Benzotriazolyl Tetramethylbutylphenol and corresponds to formula

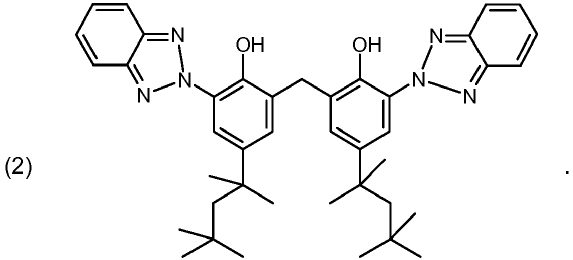

Most preferably the organic UV-filter (b₂) corresponds to the formula

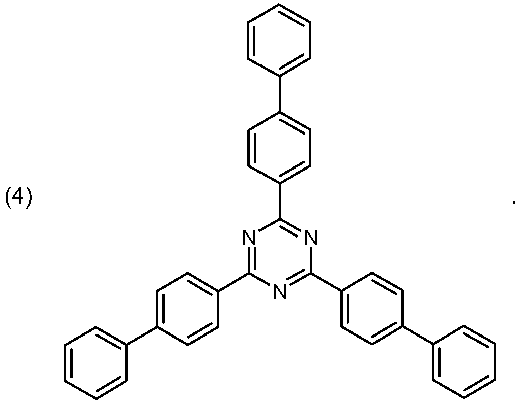

The cosmetic formulation according to the present invention preferably comprises
0.01 to 10 % b.w of particulates (a); and
0.1 to 40 % b.w of UV filters mixture (b).

The particulate organic UV-filters (b) according to the present invention are present in the micronized state. They may be prepared by any known process suitable for the preparation of microparticles, for example: wet-milling, wet-kneading, spray-drying from a suitable solvent, by expansion according to the RESS process (Rapid Expansion of Supercritical Solutions) of supercritical fluids (e.g. CO₂, by reprecipitation from suitable solvents, including supercritical fluids (GASR process = Gas Anti-Solvent Recrystallisation / PCA process = Precipitation with Compressed Anti-solvents).

As milling apparatus for the preparation of the sparingly soluble micronised organic compounds there may be used, for example, a jet mill, ball mill, vibratory mill or hammer mill, preferably a high-speed mixing mill. Even more preferable mills are modern ball mills; manufacturers of these types of mill are, for example, Netzsch (LMZ mill), Drais (DCP-Viscoflow or Cosmo), Bühler AG (centrifugal mills) or Bachhofer.

Examples of kneading apparatus for the preparation of the micronised organic UV absorbers are typical sigma-blade batch kneaders but also serial batch kneaders (IKA-Werke) or continuous kneaders (Continua from Werner und Pfleiderer).

The grinding of organic compounds used in the present invention is preferably carried out with a grinding aid.

Preferably, a dispersing agent is used as a low molecular weight grinding aid for all the above micronisation processes.

Preferred useful grinding aids for an aqueous dispersion are anionic surfactants with a HLB (Hydrophile-Lipophile Balance) value higher than 8, more preferably higher than 10.

Any conventionally usable anionic, non-ionic or amphoteric surfactants can be used as dispersing agents. Such surfactant systems may comprise for example: carboxylic acids and their salts: alkaline soap of sodium, potassium and ammonium, metallic soap of calcium or magnesium, organic basis soap such as Lauric, myristic, palmitic, stearic and oleic acid etc., alkyl phosphates or phosphoric acid esters, acid phosphate, diethanolamine phosphate, potassium cetyl phosphate, ethoxylated carboxylic acids or polyethyleneglycol esters, PEG-n acrylates, fatty alcohol polyglycolether such as laureth-n, myreth-n, ceteareth-n, steareth-n, oleth-n, fatty acid polyglycolether such as PEG-n stearate, PEG-n oleate, PEG-n cocoate, monoglycerides and polyol esters, C12-C22 fatty acid mono- and di-esters of addition products of from 1 to 100 mol of ethylene oxide with polyols,fatty acid and polyglycerol ester such as monostearate glycerol, diisostearoyl polyglyceryl-3-diisostearates, polyglyceryl-3-diisostearates, triglyceryl diisostearates, polyglyceryl-2-sesquiisostearates or polyglyceryl dimerates. mixtures of compounds from a plurality of those substance classes are also suitable, fatty acid polyglycolesters such as monostearate diethylene glycol, fatty acid and polyethylene glycol esters, fatty acid and saccharose esters such as sucro esters, glycerol and saccharose esters such as sucro glycerides. Sorbitol and sorbitan, sorbitan mono- and di-esters of saturated and unsaturated fatty acids having from 6 to 22 carbon atoms and ethylene oxide addition products, polysorbate-n series, sorbitan esters such as sesquiisostearate, sorbitan, PEG-(6)-isostearate sorbitan, PEG-(10)-sorbitan laurate, PEG-17- dioleate sorbitan, glucose derivatives, C8-C22 alkyl-mono and oligo-glycosides and ethoxylated analogues with glucose being preferred as the sugar component, O/W emulsifiers such as methyl gluceth-20 sesquistearate, sorbitan stearate/sucrose cocoate, methyl glucose sesquistearate, cetearyl alcohol/cetearyl glucoside, W/O emulsifiers such as methyl glucose dioleate/ methyl glucose isostearate. Sulfates and sulfonated derivatives, dialkylsulfosuccinates, dioctyl succinate, alkyl lauryl sulfonate, linear sulfonated parafins, sulfonated tetraproplyne sulfonate, sodium lauryl sulfates, amonium and ethanolamine lauryl sulfates, lauryl ether sulfates, sodium laureth sulfates [Texapon N70] or sodium myreth sulfates [Texapon K14S], sulfosuccinates, aceyl isothionates, alkanolamide sulfates, taurines, methyl taurines, imidazole sulfates, zwitterionic or amphoteric surfactants that carry at least one quaternary ammonium group and at least one carboxylate and/or sulfonate group in the molecule, zwitterionic surfactants that are especially suitable are betaines, such as N-alkyl-N,N-dimethylammonium glycinates, cocoalkyldimethylammonium glycinate, N-acylamino-propyl-N,N-dimethylammonium glycinates, cocoacylaminopropyldimethylammonium glycinate and 2-alkyl-3-carboxymethyl-3-hydroxyethylimidazolines each having from 8 to 18 carbon atoms in the alkyl or acyl group and also cocoacylaminoethylhydroxyethylcarboxymethylglycinate, N-alkylbetaine, N-alkylaminobetaines.

Examples of suitable mild surfactants as dispersing agents, that is to say surfactants especially well tolerated by the skin, include fatty alcohol polyglycol ether sulfates, monoglyceride sulfates, mono- and/or di-alkyl sulfosuccinates, fatty acid isethionates, fatty acid sarcosinates, fatty acid taurides, fatty acid glutamates, α-olefin sulfonates, ethercarboxylic acids, alkyl oligoglucosides, fatty acid glucamides, alkylamidobetaines and/or protein fatty acid condensation products, the latter preferably being based on wheat proteins.

Non ionic surfactants such as PEG-6 beeswax (and) PEG-6 stearate (and) polyglyceryl - 2-isostearate [Apifac], glyceryl stearate (and) PEG-100 stearate. [Arlacel 165], PEG-5 glyceryl stearate [arlatone 983 S], sorbitan oleate (and) polyglyceryl-3 ricinoleate.[Arlacel 1689], sorbitan stearate and sucrose cocoate [arlatone 2121], glyceryl stearate and laureth-23 [Cerasynth 945], cetearyl alcohol and ceteth-20 [Cetomacrogol Wax], cetearyl alcohol and colysorbate 60 and PEG-150 and stearate-20[Polawax GP 200, Polawax NF], cetearyl alcohol and cetearyl polyglucoside [Emulgade PL 1618], cetearyl alcohol and ceteareth-20 [Emulgade 1000NI, Cosmowax], cetearyl alcohol and PEG-40 castor oil [Emulgade F Special], cetearyl alcohol and PEG-40 castor oil and sodium cetearyl sulfate [Emulgade F], stearyl alcohol and steareth-7 and steareth-10 [Emulgator E 2155], cetearyl alcohol and szeareth-7 and steareth-10 [Emulsifying wax U.S.N.F], glyceryl stearate and PEG-75 stearate [Gelot 64], propylene glycol ceteth-3 acetate .[Hetester PCS], propylene glycol isoceth-3 acetate [Hetester PHA], cetearyl alcohol and ceteth-12 and oleth-12 [Lanbritol Wax N 21], PEG -6 stearate and PEG-32 stearate [Tefose 1500], PEG-6 stearate and ceteth-20 and steareth-20 [Tefose 2000], PEG-6 stearate and ceteth-20 and glyceryl stearate and steareth-20 [Tefose 2561], glyceryl stearate and ceteareth-20 [Teginacid H, C, X].

Anionic emulsifiers such as PEG-2 stearate SE, glyceryl stearate SE [Monelgine, Cutina KD], propylene glycol stearate [Tegin P], cetearyl Alcohol and Sodium cetearyl sulfate [Lanette N, Cutina LE, Crodacol GP], cetearyl alcohol and sodium lauryl sulfate [Lanette W], trilaneth-4 phopshate and glycol stearate and PEG-2 stearate [Sedefos 75], glyceryl stearate and sodium lauryl Sulfate [Teginacid Special]. Cationic acid bases such as cetearyl alcohol and cetrimonium bromide.

Most preferred dispersing agents are sodium alkyl sulfates or sodium alkyl ether sulfates, such as sodium laureth sulfate [Texapon N70 from Cognis] or sodium myreth sulfate [Texapon K14 S from Cognis].

Useful solvents are water, brine, (poly-)ethylene glycol, glycerol or cosmetically acceptable oils. Other useful solvents are disclosed below in the sections entitled "Esters of fatty acids", "Natural and synthetic triglycerides, including glyceryl esters and derivatives", "Pearlescent waxes", "Hydrocarbon oils" and "Silicones or siloxanes".

The micronised particulate organic compounds so obtained usually have an average particle size from 0.02 to 2 micrometres, preferably from 0.03 to 1.5 micrometres and more especially from 0.05 to 1.0 micrometres.

The aqueous dispersion comprising a micronized UV absorber used in the present invention generally comprises
30 - 60, preferably 35 to 55 parts of the sparingly soluble organic micronized substance;
2 - 20, preferably 3 to 10 parts of the dispersing agent;
0.1 - 1 part, preferably 0.1 to 0.5 parts of a thickening agent (for example xanthan gum); and
20 - 67.9 parts of water;

The cosmetic formulations or pharmaceutical compositions according to the present invention can also comprise one or more than one further UV filter which are different from the particulate organic UV-filters (b) as listed in Table 1:

| Table 2: Suitable UV filter substances and adjuvants which can be additionally used with the UV absorbers according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxoborn-3-ylidene)toluene-4-sulphonic acid and its salts (Mexoryl SL) | 56039-58-8 |
| 12 | Methyl N,N,N-trimethyl-4-[(4,7,7-trimethyl-3-oxobicyclo[2,2,1]hept-2-ylidene)methyllanilinium sulphate (Mexoryl SO) | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |
| 28 | Menthyl salicylate | 89-46-3 |
| 29 | 2-Ethylhexyl 2-cyano,3,3-diphenylacrylate; Octocrylene | 6197-30-4 |
| 30 | 2- ethylhexyl 4- (dimethylamino)benzoate | 21245-02-3 |
| 31 | 2- ethylhexyl 4- methoxycinnamate; Octyl Methoxy Cinnamate | 5466-77-3 |
| 32 | 2- ethylhexyl salicylate | 118-60-5 |
| 34 | 4- aminobenzoic acid | 150-13-0 |
| 35 | Benzoic acid, 4-amino-, ethyl ester, polymer with oxirane | 113010-52-9 |
| 38 | 2-phenyl-1H-benzimidazole- 5- sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2- | 147897-12-9 |
| | ylidene)methyl]phenyl]methyl]-, homopolymer | |
| 40 | Triethanolamine salicylate | 2174-16-5 |
| 41 | 3, 3'-(1,4-phenylenedimethylene)bis[7, 7-dimethyl- 2-oxo-bicyclo[2.2.1]heptane-1 methanesulfonic acid] (Cibafast H) | 90457-82-2 |
| 42 | Titanium dioxide (primary particle size 10 - 50 nm); for example T805 or Eusolex T-AVO, Eusolex T-2000, Titaniumdioxid VT 817 | 13463-67-7 |
| 44 | Zinc oxide (primary particle size 20-100 nm); for example Zinc oxide NDM, Zinc oxide Z-Cote HP1, Nanox Zinc oxide | 1314-13-2 |
| 47 | 1H-Benzimidazole-4,6-disulfonic acid, 2,2'-(1,4-phenylene)bis-, disodium salt | 180898-37-7 |
| 48 | Benzoic acid, 4,4'-[[6-[[4-[[(1,1-dimethylethyl)amino]carbonyl]-phenyl]amino]1,3,5-triazine-2,4-diyl]diimino]bis-, bis(2-ethylhexyl)-ester; diethylhexyl butamido triazone (Uvasorb HEB) | 154702-15-5 |
| 49 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane (Mexoryl XL) | 155633-54-8 |
| 50 | Dimethicodiethylbenzalmalonate; Polysilicone 15 (Parsol SLX) | 207574-74-1 |
| 51 | Benzenesulfonic acid, 3-(2H-benzotriazol-2-yl)-4-hydroxy-5-(1-methylpropyl)-, monosodium salt (Tinogard HS) | 92484-48-5 |
| 53 | 1-Dodecanaminium, N-[3-[[4-(dimethylamino)benzoyl]amino]propyl]-N,N-dimethyl-, salt with 4-methylbenzenesulfonic acid (1:1) (Escalol HP610) | 156679-41-3 |
| 54 | 1-Propanaminium, N,N,N-trimethyl-3-[(1-oxo-3-phenyl-2-propenyl)-amino]-, chloride | 177190-98-6 |
| 55 | 1H-Benzimidazole-4,6-disulfonic acid, 2,2'-(1,4-phenylene)bis- | 170864-82-1 |
| 56 | 1,3,5-Triazine, 2,4,6-tris(4-methoxyphenyl)- | 7753-12-0 |
| 57 | 1,3,5-Triazine, 2,4,6-tris[4-[(2-ethylhexyl)oxy]phenyl]- | 208114-14-1 |
| 58 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 59 | 2-Propenoic acid, 3-(1H-imidazol-4-yl)- | 104-98-3 |
| 60 | Benzoic acid, 2-hydroxy-, [4-(1-methylethyl)phenyl]methyl ester | 94134-93-7 |
| 61 | 1,2,3-Propanetriol, 1-(4-aminobenzoate) (Glyceryl PABA) | 136-44-7 |
| 62 | Benzeneacetic acid, 3,4-dimethoxy-a-oxo- | 4732-70-1 |
| 63 | 2-Propenoic acid, 2-cyano-3,3-diphenyl-, ethyl ester | 5232-99-5 |
| 64 | Anthralinic acid, p-menth-3-yl ester | 134-09-8 |
| 65 | 2,2'-bis(1,4-phenylene)-1H-benzimidazole-4,6-disulphonic acid mono sodium salt or Disodium phenyl dibenzimidazole tetrasulfonate (Neo Heliopan AP) | 349580-12-7 |
| 69 | sterols (cholesterol, lanosterol, phytosterols), as described in WO0341675 | |
| 70 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes,) | |
| 71 | alpha-lipoic-acid as described in DE 10229995 | |
| 72 | synthetic organic polymers as described in EP 1 371 358, [0033]-[0041] | |
| 73 | phyllosilicates as described in EP 1371357 [0034]-[0037] | |
| 74 | silica compounds as described in EP1371356, [0033]-[0041] | |
| 75 | inorganic particles as described in DE10138496 [0043]-[0055] | |
| 76 | latex particles as described in DE10138496 [0027]-[0040] | |
| 77 | 1H-Benzimidazole-4,6-disulfonic acid, 2,2'-(1,4-phenylene)bis-, disodium salt; Bisimidazylate (Neo Heliopan APC) | 180898-37-7 |
| 82 | Di-2-ethylhexyl-3,5-dimethoxy-4-hydroxy-benzalmalonate (Oxynex ST, EMD Chemicals, as described in US 20040247536) | |
| 83 | T-LiteTM MAX: Titanium Dioxide (and) Dimethoxydiphenylsilane (and) Triethoxycaprylylsilane Crosspolymer (and) Hydrated Silica (and) Aluminum Hydroxyde | |
| 84 | T-Lite SF: Titanium Dioxide (and) Aluminum Hydroxide (and) Dimethicone/Methicone Copolymer | |
| 85 | T-Lite SF-S: Titanium Dioxide (and) Hydrated Silica (and) Dimethicone/ Methicone Copolymer (and) Aluminum Hydroxide | |
| 86 | Z-COTE® MAX: Zinc Oxide (and) Diphenyl Capryl Methicone | |
| 87 | Z-COTE HP1: Zinc Oxide (and) Triethoxycaprylylsilane | |
| 88 | 1,3,5-Triazine-2,4,6-triamine, N2,N4-bis[4-[5-(1,1-dimethylpropyl)-2-benzoxazolyl]phenyl]-N6-(2-ethylhexyl)- (Uvasorb K2A) | 288254-16-0 |

The cosmetic or pharmaceutical preparations can be prepared by physically mixing the UV absorber(s) with the adjuvant using customary methods, for example by simply stirring together the individual components, especially by making use of the dissolution properties of already known cosmetic UV absorbers, like octyl methoxy cinnamate, salicylic acid isooctyl ester, etc.

The cosmetic or pharmaceutical preparations may be, for example, creams, gels, lotions, alcoholic and aqueous/alcoholic solutions, emulsions, wax/fat compositions, stick preparations, powders or ointments. In addition to the above mentioned UV filters, the cosmetic or pharmaceutical preparations may contain further adjuvants as described below.

As water- and oil-containing emulsions (e.g. W/O, O/W, O/W/O and W/O/W emulsions or microemulsions) the preparations contain, for example, from 0.1 to 30 % by weight, preferably from 0.1 to 15 % by weight and especially from 0.5 to 10 % by weight, based on the total weight of the composition, of one or more UV absorbers, from 1 to 60 % by weight, especially from 5 to 50 % by weight and preferably from 10 to 35 % by weight, based on the total weight of the composition, of at least one oil component, from 0 to 30 % by weight, especially from 1 to 30 % by weight und preferably from 4 to 20 % by weight, based on the total weight of the composition, of at least one emulsifier, from 10 to 90 % by weight, especially from 30 to 90 % by weight, based on the total weight of the composition, of water, and from 0 to 88.9 % by weight, especially from 1 to 50 % by weight, of further cosmetically acceptable adjuvants.

The cosmetic or pharmaceutical compositions/preparations according to the invention may also contain one or one more additional compounds as like fatty alcohols, esters of fatty acids, natural or synthetic triglycerides including glyceryl esters and derivatives, pearlescent waxes, hydrocarbon oils, silicones or siloxanes (organosubstituted polysiloxanes), fluorinated or perfluorinated oils, emulsifiers, djuvants and additives, super-fatting agents, surfactants, consistency regulators/thickeners and rheology modifiers, polymers, biogenic active ingredients, deodorising active ingredients, anti-dandruff agents, antioxidants, hydrotropic agents, preservatives, bacteria-inhibiting agents, perfume oils, colourants, polymeric beads or hollow spheres as SPF enhancers.

### Cosmetic or pharmaceutical preparations

Cosmetic or pharmaceutical formulations are contained in a wide variety of cosmetic preparations. There come into consideration, for example, especially the following preparations: skin-care preparations, bath preparations, cosmetic personal care preparations, foot-care preparations, light-protective preparations, skin-tanning preparations, depigmenting preparations, insect-repellents, deodorants, antiperspirants, preparations for cleansing and caring for blemished skin, hair-removal preparations in chemical form (depilation), shaving preparations, fragrance preparations, cosmetic hair-treatment preparations,

### Presentation forms

The final formulations listed may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a powder, a lacquer, a tablet or make-up,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray.

Of special importance as cosmetic preparations for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

Other typical ingredients in such formulations are preservatives, bactericides and bacteriostatic agents, perfumes, dyes, pigments, thickening agents, moisturizing agents, humectants, fats, oils, waxes or other typical ingredients of cosmetic and personal care formulations such as alcohols, poly-alcohols, polymers, electrolytes, organic solvents, silicon derivatives, emollients, emulsifiers or emulsifying surfactants, surfactants, dispersing agents, antioxidants, anti-irritants and anti-inflammatory agents etc.

Preferably particulates (a) are used which can easily be removed from textiles in customary laundering processes.

Other embodiments of the present invention are the use of the cosmetic formulation according to the present invention
- for minimizing and/or masking the whitening effect and/or the particular color of a sunscreen composition;
- for the visualization of a consistent application of a sunscreen formulation;
- for adjusting a natural skin-like color tone of the sunscreen formulation;
- for promoting an even skin tone; and
- for minimising fine lines and wrinkles.

The composition is used for preventing the damaging effect of UVA and blue light and provides an optimal protection against UVA-induced photoaging of human skin also in conjunction with excessive exposure to longer wave-length light and IR.

The cosmetic compositions according to the present invention may also be used for preventing and treating undesired skin pigmentation; preventing and treating photoaging of human skin; inhibiting the degradation or condensations of endogenous vitamins, antioxidants and cellular modulators such as carotenoids, flavonoids or unsaturated lipids present in the skin; treating skin disorders caused by sunlight such as pigment spots, actinic keratosis and actinic dermatitis and solar urticaria; protecting against UVA1-induced expression of matrix-degrading enzymes such as metallo-proteinases or elastases, e.g. metallo-proteinase-1 or neutrophil elastase or of cytokines such as interleukin-1 or interleukin-6; preventing the generation of reactive oxygen species such as by photosensitizing molecules, via UV-induced inflammatory reactions or via impaiment of mitochondria function; protecting UV-A-sensitive pharmaceuticals and cosmetics.

The compositions according to the present invention may constitute a skin care product, in particular for the face, neck, contours of the eye or the body, or a skin makeup product such as a tinting product, an eye shadow, a blusher, an eyeliner, a concealer, a body makeup product, a daily care product, a sun protection product or a skin cleansing product.

The cosmetic preparation according to the invention is distinguished by excellent protection of human skin against the damaging effect of sunlight.

### Examples

### Preparation of Sunscreen Formulation

Examples 1, 2 and 4 show advantage over Examples 3 and 5 which do not contain color pigments.

| INCI | Ex. 1 | Ex. 2 | Ex. 3 |
|---|---|---|---|
| Part A | | | |
| Glyceryl Stearate (and) PEG 100 Stearate | 2.00 | 2.00 | 2.00 |
| Butylene Glycol Cocoate | 5.00 | 5.00 | 5.00 |
| Isopropyl Palmitate | 4.00 | 4.00 | 4.00 |
| Trioctanion | 4.00 | 4.00 | 4.00 |
| TiO₂ (and) Stearic Acid (and) Aluminium Hydroxide | 3.20 | 3.20 | 3.20 |
| Part B | | | |
| Water | | | |
| Tinoderm SGP (Ciba) | 3.00 | 3.00 | 3.00 |
| Disodium EDTA | 0.20 | 0.20 | 0.20 |
| Xanthan Gum | 0.20 | 0.20 | 0.20 |
| Potassium cetyl Phosphate | 1.50 | 1.50 | 1.50 |
| Part B2 | | | |
| Calisha Contemporary jet black (Iron oxide) | 0.01 | 0.005 | - |
| Calisha Contemporary medium red (iron oxide) | 0.1 | 0.025 | - |
| Calisha Contemporary yellow (iron oxide) | 0.55 | 0.27 | - |
| Part C | | | |
| Phenyl Trimethicone | 2.00 | 2.00 | 2.00 |
| Cyclomethicone | 4.00 | 4.00 | 4.00 |
| Dimethicone (and) Dimethicone/Vinyl Dimethicone Crosspolymer | 3.00 | 3.00 | 3.00 |
| Part D | | | |
| Mica (and) Titanium Dioxide (and) Red iron oxide (and) Cetyl Dimethicone | 0.30 | | |
| Micronized Methylene Bis-Benzotriazolyl Tetramethylbutylphenol | 4.00 | 5.00 | 3.00 |
| Micronized 1,3,5-Triazine, 2,4,6-tris([1,1'-biphenyl]-4-yl)- (CAS Regn. 31274-51-8) | 5.00 | 5.00 | 5.00 |
| Phenoxyethanol (and) Methylparaben (and) Ethylparaben (and) Butylparaben (and) Propylparaben Isobutylparaben | 1.00 | | |
| Tinoderm E | | | 0.50 |
| Water | ad 100 | ad 100 | ad 100 |

### Manufacturing process:

Heat up part A and B to 75°C.
Add Amphisol K into B and stirr for 5 more minutes, then Add part B2.
Add part A into part B, then homogenize with an Ultra Turrax for 1 min pos 2/200g
Add part C by 60°C, and turrax again
Add part D below 50°C

### B. Application Examples

| Example B1: O/W Emulsion (1) | | | | |
|---|---|---|---|---|
| Part | INCI | A | B | C |
| A | Glyceryl Stearate (and) PEG 100 Stearate | 2.00 | 2.00 | 2.00 |
| | Butylene Glycol Cocoate | 5.00 | 5.00 | 5.00 |
| | Isopropyl Palmitate | 4.00 | 4.00 | 4.00 |
| | Triethylhexanoin | 4.00 | 4.00 | 4.00 |
| | TIO2 (and) Stearic Acid (and) Aluminum Hydroxide | 3.20 | 3.20 | 3.20 |
| B | | | | |
| | Pentylene Glycol (and) Sclerotium Gum | 3.00 | 3.00 | 3.00 |
| | Disodium EDTA | 0.20 | 0.20 | 0.20 |
| | Xanthan Gum | 0.20 | 0.24 | 0.24 |
| | Potassium cetyl Phosphate | 1.50 | 1.50 | 1.50 |
| B 2 | Iron Oxides (CALISHA Contemprorary Jet Black Oxide) | 0.01 | 0.005 | 0.005 |
| | Iron Oxides (CALISHA Contemporary Medium Red Oxide) | 0.10 | 0.025 | 0.025 |
| | Iron Oxides (CALISHA Contemporary Yellow Oxide) | 0.55 | 0.27 | 0.27 |
| C | Phenyl Trimethicone | 2.00 | 2.00 | 2.00 |
| | Cyclomethicone | 4.00 | 4.00 | 4.00 |
| | Dimethicone (and) Dimethicone/Vinyl Dimethicone Crosspolymer | 3.00 | 3.00 | 3.00 |
| D | Micronized Methylene Bis-Benzotriazolyl Tetramethylbutylphenol | 4.00 | 4.00 | 4.00 |
| | Micronized 1,3,5-Triazine, 2,4,6-tris([1,1'-biphenyl]-4-yl)-(CAS Regn. 31274-51-8) | 5.00 | 5.00 | 5.00 |
| | Micronized Methanone, 1,1'-(1,4-piperazinediyl)bis[1-[2-[4-(diethylamino)-2-hydroxybenzoyl]phenyl]-(CAS Regn. 919803-06-8) | 1.00 | 2.00 | 4.00 |
| | Mica (and) Titanium Dioxide (and)Tin oxide | 0.30 | | 0.30 |
| | Phenoxyethanol (and) Methylparaben (and) Ethylparaben (and) Butylparaben (and) Propylparaben (and) Isobutylparaben | 1.00 | 1.00 | 1.00 |
| | Aqua (and) Tocopheryl Acetate (and) Caprylic/Capric Triglyceride (and) Polysorbate 80 (and) Lecithin | | | 0.50 |
| | Water | ad 100 | ad 100 | ad 100 |

| Example B2: O/W Emulsion (2) | | | | | | |
|---|---|---|---|---|---|---|
| INCI | A | B | C | D | E | F |
| Glyceryl Stearate (and) PEG 100 Stearate | 5.00 | 5.00 | 5.00 | 5.00 | 5.00 | 5.00 |
| Phenethyl Benzoate | 6.00 | 6.00 | 6.00 | 6.00 | 6.00 | 6.00 |
| Isopropyl Palmitate | 5.00 | 5.00 | 5.00 | 5.00 | 5.00 | 5.00 |
| Triethylhexanoin | 3.00 | 3.00 | 3.00 | 3.00 | 3.00 | 3.00 |
| Bis-Ethylhexyloxyphenol Methoxyphenyl Triazine | 2.00 | 2.00 | 2.00 | 2.00 | 2.00 | 2.00 |
| Ethylhexyl Triazone | 1.50 | 1.50 | 1.50 | 1.50 | 1.50 | 1.50 |
| Butylene Glycol | 2.00 | 2.00 | 2.00 | 2.00 | 2.00 | 2.00 |
| Disodium EDTA | 0.20 | 0.20 | 0.20 | 0.20 | 0.20 | 0.20 |
| Iron Oxides (CALISHA Contemprorary Jet Black Oxide) | 0.01 | 0.02 | 0.01 | 0.01 | 0.01 | 0.01 |
| Iron Oxides (CALISHA Contemporary Medium Red Oxide) | 0.08 | 0.5 | 0.08 | 0.08 | 0.08 | 0.08 |
| Iron Oxides (CALISHA Contemporary Yellow Oxide | 0.55 | 0.5 | 0.55 | 0.55 | 0.55 | 0.55 |
| Sodium Acrylates Copolymer (and) Mineral Oil (and) PPG-1 Trideceth-6 | 1.20 | 1.20 | 1.20 | 1.20 | 1.20 | 1.20 |
| Cyclomethicone | 4.00 | 4.00 | 4.00 | 4.00 | 4.00 | 4.00 |
| Micronized Methylene Bis-Benzotriazolyl Tetramethylbutylphenol | 4.00 | 4.00 | 4.00 | 1.00 | 2.00 | 2.00 |
| Butyl Methoxydibenzoymethane | 0.5 | | 4.00 | 4.00 | | 0.5 |
| Ethylhexyl Methoxycinnamate | 0.5 | | 3.00 | 3.00 | | 0.5 |
| Octocrylene | 0.5 | | 4.00 | 4.00 | | 0.5 |
| Propanedinitrile, 2-[3-[bis(2-ethylhexyl)-amino]-2-cyclohexen-1-ylidene]- (CAS Regn. 923271-36-7) or merocyanine derivative of formula (14) or 1,3,5-Triazine-2,4,6-triamine, N2,N4-bis[4-[5-(1,1-dimethylpropyl)-2-benzoxazolyl]phenyl]-N6-(2-ethylhexyl)- (Uvasorb K2A) (CAS Regn 288254-16-0) | | | | 2.00 | | 0.5 |
| Acetic acid, 2-cyano-2-[5,5-dimethyl-3-[(1-methylpropyl)amino]-2-cyclohexen-1-ylidene]-, 2-ethylhexyl ester (CAS Regn. 923271-35-6) | | 2.00 | | | 1.00 | 0.5 |
| Uvinul A plus (CAS Regn. 302776-68-7) | 0.5 | 0.5 | 2.00 | | | 2.00 |
| Micronized 1,3,5-Triazine, 2,4,6-tris([1,1'-biphenyl]-4-yl)- (CAS Regn. 31274-51-8) | 2.00 | 2.00 | 2.00 | 2.00 | 2.00 | 2.00 |
| 2,4-Pentadienoic acid, 5-(diethylamino)-2-(phenylsulfonyl)-, octyl ester (CAS Regn. 98835-90-6) | 2.00 | | 0.5 | 0.5 | 0.5 | 0.5 |
| Micronized Methanone, 1,1'-(1,4-piperazine-diyl)bis[1-[2-[4-(diethylamino)-2-hydroxybenzoyl]phenyl]- (CAS Regn. 919803-06-8) | 2.00 | 2.00 | 2.00 | 2.00 | | 2.00 |
| Mica (and) Titanium Dioxide (and)Tin oxide | 1.00 | 1.00 | 1.00 | 1.00 | 1.00 | 1.00 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 | 1.00 |
| Water | ad 100 | ad 100 | ad 100 | ad 100 | ad 100 | ad 100 |

| Example B3: O/W Emulsion (3) | | | | | | |
|---|---|---|---|---|---|---|
| INCI | A | B | C | D | E | F |
| Tribehenin PEG-20 esters | 5.00 | 5.00 | 5.00 | 5.00 | 5.00 | 5.00 |
| Stearyl Alcohol | 0.50 | 0.50 | 0.50 | 0.50 | 0.50 | 0.50 |
| Dibutyl Adipate | 5.00 | 5.00 | 5.00 | 5.00 | 5.00 | 5.00 |
| PPG-2 Myristyl Ether Propionate | 5.00 | 5.00 | 5.00 | 5.00 | 5.00 | 5.00 |
| Triheptanoin | 3.00 | 3.00 | 3.00 | 3.00 | 3.00 | 3.00 |
| Ethylhexyl Salicylate | 5.00 | 5.00 | 5.00 | 5.00 | 5.00 | 5.00 |
| Butyl Methoxydibenzoymethane | 3.00 | 3.00 | 3.00 | 3.00 | 3.00 | 3.00 |
| Bis-Ethylhexyloxyphenol Methoxyphenyl Triazine | 3.00 | 3.00 | 3.00 | 3.00 | 3.00 | 3.00 |
| Ethylhexyl Triazone | 3.00 | 3.00 | 3.00 | 3.00 | 3.00 | 3.00 |
| Micronized Methylene Bis-Benzotriazolyl Tetramethylbutylphenol | 2.00 | 2.00 | 2.00 | 2.00 | 2.00 | 2.00 |
| Butyl Methoxydibenzoymethane | | 4.00 | | 0.5 | 0.5 | 0.5 |
| Bis-Ethylhexyloxyphenol Methoxyphenyl Triazine | 1.00 | 1.00 | 1.00 | 1.00 | 1.00 | 1.00 |
| Ethylhexyl Triazone | 0.5 | 0.5 | 0.5 | 0.5 | 0.5 | 0.5 |
| Ethylhexyl Methoxycinnamate | | 4.00 | | | | |
| Octocrylene | | 4.00 | | 0.5 | 0.5 | 0.5 |
| Propanedinitrile, 2-[3-[bis(2-ethylhexyl)amino]-2-cyclohexen-1-ylidene]-(CAS Regn. 923271-36-7) or merocyanine derivative of formula (14) or 1,3,5-Triazine-2,4,6-triamine, N2,N4-bis[4-[5-(1,1-dimethylpropyl)-2-benzoxazolyl]-phenyl]-N6-(2-ethylhexyl)-(Uvasorb K2A) (CAS Regn 288254-16-0) | 2.00 | | 0.5 | | 1.0 | |
| Acetic acid, 2-cyano-2-[5,5-dimethyl-3-[(1-methylpropyl)amino]-2-cyclohexen-1-ylidene]-, 2-ethylhexyl ester (CAS Regn. 923271-35-6) | | | 2.00 | | | |
| Uvinul A plus (CAS Regn. 302776-68-7) | | | | 2.00 | | |
| Micronized 1,3,5-Triazine, 2,4,6-tris-([1,1'-biphenyl]-4-yl)- (CAS Regn. 31274-51-8) | 2.00 | 5.00 | 2.00 | 2.00 | 2.00 | 4.00 |
| 2,4-Pentadienoic acid, 5-(diethylamino)-2-(phenylsulfonyl)-, octyl ester (CAS Regn. 98835-90-6) | | 2.00 | | | | |
| Micronized Methanone, 1,1'-(1,4-piperazinediyl)bis[1-[2-[4-(diethylamino)-2-hydroxybenzoyl]phenyl]-(CAS Regn. 919803-06-8) | 2.00 | 2.00 | 5.00 | 4.00 | 2.00 | 2.00 |
| Sodium Polyacrylate | | 0.60 | 0.60 | 0.60 | 0.60 | 0.60 |
| Disodium EDTA | 0.20 | 0.20 | 0.20 | 0.20 | 0.20 | 0.20 |
| Xanthan Gum | 0.50 | | 0.50 | 0.50 | 0.50 | 0.50 |
| Iron Oxides (CALISHA Contemprorary Black Oxide) | 0.020 | 0.020 | 0.020 | 0.020 | 0.020 | 0.020 |
| Iron Oxides (CALISHA Contemporary Salmon Red) | 0.08 | | 0.08 | 0.08 | 0.08 | 0.08 |
| Iron Oxides (CALISHA Contemporary Sun Yellow) | 0.55 | | 0.55 | 0.55 | 0.55 | 0.55 |
| Cyclomethicone | 5.00 | 5.00 | 5.00 | 5.00 | 5.00 | 5.00 |
| Mica (and) Titanium Dioxide (and) Iron Oxides (and) Cetyl Dimethicone | 0.50 | 0.50 | 0.50 | 0.50 | 0.50 | 0.50 |
| Mica (and) Titanium Dioxide (and) Cetyl Dimethicone (and) Iron Oxides (and) Carmine | 0.50 | 0.50 | 0.50 | 0.50 | 0.50 | 0.50 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 | 1.00 |
| Water | ad 100 | ad 100 | ad 100 | ad 100 | ad 100 | ad 100 |

| Example B4: W/O Emulsion (1) | | | | | |
|---|---|---|---|---|---|
| INCI | A | B | C | D | E |
| Polyglyceryl -4 Isostearate (and) Cetyl PEG/PPG-10/1 Dimethicone (and) Hexyl Laurate | 4.50 | 4.50 | 4.50 | 4.50 | 4.50 |
| Cetyl PEG/PPG-10/1 Dimethicone | 2.20 | 2.20 | 2.20 | 2.20 | 2.20 |
| Sorbitan Sesquioleate | 1.00 | 1.00 | 1.00 | 1.00 | 1.00 |
| Cera Alba | 1.80 | 1.80 | 1.80 | 1.80 | 1.80 |
| Hydrogenated Vegetable Oil | 2.00 | 2.00 | 2.00 | 2.00 | 2.00 |
| Phenethyl Benzoate | 2.00 | 2.00 | 2.00 | 2.00 | 2.00 |
| Bis-Ethylhexyloxyphenol Methoxyphenyl Triazine | 1.00 | 1.00 | 3.00 | 1.00 | 1.00 |
| Ethylhexyl Methoxycinnamate | 5.00 | 5.00 | 5.00 | 10.00 | 5.00 |
| Titanium Dioxide (and) Silica (and) Dimethicone | 5.00 | 5.00 | 5.00 | 5.00 | 5.00 |
| Iron Oxides (and) Cetyl Dimethicone | 0.13 | 0.13 | 0.13 | 0.13 | 0.13 |
| Iron Oxides (and) Cetyl Dimethicone | 0.60 | 0.60 | 0.60 | 0.60 | 0.60 |
| Iron Oxides (and) Cetyl Dimethicone | 0.20 | 0.20 | 0.20 | 0.20 | 0.20 |
| Cyclomethicone (and) Disteardimonium Hectorite (and) Propylene Carbonate | 5.00 | 5.00 | 5.00 | 5.00 | 5.00 |
| Cyclopentasiloxane | 6.00 | 6.00 | 6.00 | 6.00 | 6.00 |
| Ethyl Trisiloxane | 8.00 | 8.00 | 8.00 | 8.00 | 8.00 |
| Dimethicone | 6.00 | 6.00 | 6.00 | 6.00 | 6.00 |
| Cyclopentasiloxane (and) C30-45 Alkyl Cetearyl Dimethicone Crosspolymer (and) PEG/PPG-20/23 Dimethicone | 1.00 | 1.00 | 1.00 | 1.00 | 1.00 |
| Water | ad 100 | ad 100 | ad 100 | ad 100 | ad 100 |
| Sodium Chloride | 1.00 | 1.00 | 1.00 | 1.00 | 1.00 |
| Pentylene Glycol (and) Scerotium Gum | 3.00 | 3.00 | 3.00 | 3.00 | 3.00 |
| Aqua (and) Retinyl Palmitate (and) Capryic/Capric Triglyceride (and) Polysorbate 80 (and) Lecithin (and) Benzalkonium Chloride | 3.00 | 3.00 | 3.00 | 3.00 | 3.00 |
| Butylen Glycol | 3.00 | 3.00 | 3.00 | 3.00 | 3.00 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 |
| Aluminum Starch Octenylsuccinate | 2.00 | 2.00 | 2.00 | 2.00 | 2.00 |
| Talc | 2.00 | 2.00 | 2.00 | 2.00 | 2.00 |
| Silica | 5.00 | 5.00 | 5.00 | 5.00 | 5.00 |
| Mica (and) Iron Oxides (and) Cetyl Dimethicone | 2.00 | 2.00 | 2.00 | 2.00 | 2.00 |
| Mica (and) Titanium Dioxide (and) Iron Oxides (and) Cetyl Dimethicone | | 1 | | 0.5 | |
| Micronized Methylene Bis-Benzotriazolyl Tetramethylbutylphenol | 0.5 | 6.00 | 4.00 | 2.00 | 1.00 |
| Butyl Methoxydibenzoymethane | 5.00 | 5.00 | 5.00 | 5.00 | 5.00 |
| Ethylhexyl Triazone | 0.60 | 0.60 | 0.60 | 0.60 | 0.60 |
| Octocrylene | 4.00 | 0.5 | 0.5 | 0.5 | 0.5 |
| Propanedinitrile, 2-[3-[bis(2-ethylhexyl)amino]-2-cyclohexen-1-ylidene]- (CAS Regn. 923271-36-7) or merocyanine derivative of formula (14) or 1,3,5-Triazine-2,4,6-triamine, N2,N4-bis[4-[5-(1,1-dimethylpropyl)-2-benzoxazolyl]phenyl]-N6-(2-ethylhexyl)-(Uvasorb K2A) (CAS Regn 288254-16-0) | 2.00 | 1.00 | 5.00 | 2.00 | 1.00 |
| Acetic acid, 2-cyano-2-[5,5-dimethyl-3-[(1-methylpropyl)amino]-2-cyclohexen-1-ylidene]-, 2-ethylhexyl ester (CAS Regn. 923271-35-6) | 6.00 | 6.00 | 6.00 | 6.00 | 6.00 |
| Uvinul A plus (CAS Regn. 302776-68-7) | 8.00 | 8.00 | 8.00 | 8.00 | 8.00 |
| Micronized 1,3,5-Triazine, 2,4,6-tris([1,1'-biphenyl]-4-yl)- (CAS Regn. 31274-51-8) | 6.00 | 6.00 | 6.00 | 6.00 | 6.00 |
| 2,4-Pentadienoic acid, 5-(diethylamino)-2-(phenylsulfonyl)-, octyl ester (CAS Regn. 98835-90-6) | 1.00 | 1.00 | 1.00 | 1.00 | 1.00 |
| Micronized Methanone, 1,1'-(1,4-piperazine-diyl)bis[1-[2-[4-(diethylamino)-2-hydroxybenzoyl]-phenyl]-(CAS Regn. 919803-06-8) | 1.00 | 3.00 | 2.00 | 4.00 | 0.5 |

| Example B5: W/O Emulsion (2) | | | | | |
|---|---|---|---|---|---|
| INCI | A | B | C | D | E |
| PEG-30 Dipolyhydroxystearate | 3.00 | 3.00 | 3.00 | 3.00 | 3.00 |
| Butylen Glycol Cocoate | 2.00 | 2.00 | 2.00 | 2.00 | 2.00 |
| Isodecyl neopentanoate | 4.00 | 4.00 | 4.00 | 4.00 | 4.00 |
| Isopropyl Isosterate | 3.00 | 3.00 | 3.00 | 3.00 | 3.00 |
| Beeswax | 1.00 | 1.00 | 1.00 | 1.00 | 1.00 |
| Hydrogenated Castor Oil | 0.80 | 0.80 | 0.80 | 0.80 | 0.80 |
| Ethylhexyl Methoxycinnamate | 5.00 | 5.00 | 5.00 | 5.00 | 5.00 |
| Iron Oxides (and) Cetyl Dimethicone (Calisha Contemporary Black Oxide OD) | 0.10 | 0.10 | 0.10 | 0.10 | 0.10 |
| Iron Oxides (and) Cetyl Dimethicone (Calisha Contemporary Yellow Oxide OD) | 0.57 | 0.57 | 0.57 | 0.57 | 0.57 |
| Iron Oxides (and) Cetyl Dimethicone (Calisha Contemporary Medium Red Oxide OD) | 0.13 | 0.13 | 0.13 | 0.13 | 0.13 |
| Caprylyl Methicone | 2.50 | 2.50 | 2.50 | 2.50 | 2.50 |
| Water | ad 100 | ad 100 | ad 100 | ad 100 | ad 100 |
| Sodium Chloride | 1.00 | 1.00 | 1.00 | 1.00 | 1.00 |
| Pentylene Glycol (and) Scerotium Gum | 3.00 | 3.00 | 3.00 | 3.00 | 3.00 |
| Aqua (and) Retinyl Palmitate (and) Caprylic/Capric Triglyceride (and) Polysorbate 80 (and) Lecithin (and) Benzalkonium Chloride | 3.00 | 3.00 | 3.00 | 3.00 | 3.00 |
| Butylen Glycol | 5.00 | 5.00 | 5.00 | 5.00 | 5.00 |
| Polysorbate 80 | 0.25 | 0.25 | 0.25 | 0.25 | 0.25 |
| Caprylyl Methicone | 5.50 | 5.50 | 5.50 | 5.50 | 5.50 |
| Dimethicone | 7.00 | 7.00 | 7.00 | 7.00 | 7.00 |
| Micronized Methylene Bis-Benzotriazolyl Tetramethylbutylphenol | 3.00 | 6.00 | 3.00 | 6.00 | 2.00 |
| Phenoxyethanol (and) Methylparaben (and) Ethylparaben (and) Butylparaben (and) Propylparaben (and) Isobutylparaben | 1.00 | 1.00 | 1.00 | 1.00 | 1.00 |
| Silica | 3.00 | 3.00 | 3.00 | 3.00 | 3.00 |
| Nylon-12 | 6.00 | 6.00 | 6.00 | 6.00 | 6.00 |
| Bis-Ethylhexyloxyphenol Methoxyphenyl Triazine | 1.00 | 1.00 | 1.00 | 1.00 | 1.00 |
| Butyl Methoxydibenzoymethane | 5.00 | 5.00 | 5.00 | 5.00 | 5.00 |
| Bis-Ethylhexyloxyphenol Methoxyphenyl Triazine | 0.13 | 0.13 | 0.13 | 0.13 | 0.13 |
| Ethylhexyl Triazone | 0.60 | 0.60 | 0.60 | 0.60 | 0.60 |
| Octocrylene | 4.00 | 0.5 | 0.5 | 0.5 | 0.5 |
| Propanedinitrile, 2-[3-[bis(2-ethylhexyl)amino]-2-cyclohexen-1-ylidene]- (CAS Regn. 923271-36-7) or merocyanine derivative of formula (14) or 1,3,5-Triazine-2,4,6-triamine, N2,N4-bis[4-[5- | 2.00 | 1.00 | 5.00 | 2.00 | 1.00 |
| (1,1-dimethylpropyl)-2-benzoxazolyl]phenyl]-N6-(2-ethylhexyl)- (Uvasorb K2A) (CAS Regn 288254-16-0 | | | | | |
| Acetic acid, 2-cyano-2-[5,5-dimethyl-3-[(1-methylpropyl)amino]-2-cyclohexen-1-ylidene]-, 2-ethylhexyl ester (CAS Regn. 923271-35-6) | 6.00 | 6.00 | 6.00 | 6.00 | 6.00 |
| Uvinul A plus (CAS Regn. 302776-68-7) | 8.00 | 8.00 | 8.00 | 8.00 | 8.00 |
| Micronized 1,3,5-Triazine, 2,4,6-tris([1,1'-biphenyl]-4-yl)- (CAS Regn. 31274-51-8) | 6.00 | 6.00 | 6.00 | 6.00 | 6.00 |
| 2,4-Pentadienoic acid, 5-(diethylamino)-2-(phenylsulfonyl)-, octyl ester (CAS Regn. 98835-90-6) | 1.00 | 1.00 | 1.00 | 1.00 | 1.00 |
| Micronized Methanone, 1,1'-(1,4-piperazine-diyl)bis[1-[2-[4-(diethylamino)-2-hydroxybenzoyl]phenyl]- (CAS Regn. 919803-06-8) | 1.00 | 3.00 | 2.00 | 4.00 | 0.5 |
| (Manganese modified Titanium Dioxide | | 4.00 | | 1.00 | |
| Diethylhexyl Syringylidene Malonate | 2.00 | 1.00 | 3.00 | 4.00 | 0.5 |
| Propanedioic acid, 2-[(4-hydroxy-3,5-dimethoxyphenyl)methyl]-, 1,3-bis(2-ethylhexyl) ester; (CAS Regn. 872182-46-2) | 1.00 | | 1.00 | | 5.00 |

## Claims

1. Cosmetic formulation comprising
(a) particulates having an absorption in the range of 400 to 800nm selected from (a₁) interference-pigments based on mica and coated with iron oxide; and
(b) UV filters selected from a mixture of particulate organic UV filters selected from the compounds of formula (1) 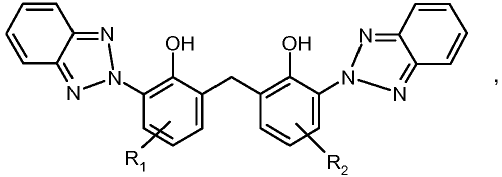 wherein
R₁ and R₂ independently from each other are C₁-C₁₈alkyl which may be substituted by one or more radicals selected from C₁-C₄alkyl, C₅-C₁₂cycloalkyl and C₆-C₁₀aryl; and the compounds of formula 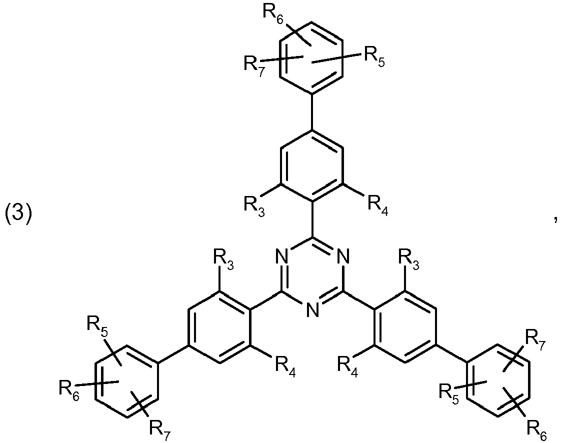 wherein
R₃ and R₄ independently from each other are hydrogen; C₁-C₁₈alkyl; or C₆-C₁₂aryl;
R₅, R₆ and R₇ independently from each other are hydrogen; hydroxy; halogen; C₁-C₁₈alkyl; C₁-C₁₈alkoxy; C₆-C₁₂aryl; biphenylyl; C₆-C₁₂aryloxy; C₁-C₁₈alkylthio; carboxy; -COOM; C₁-C₁₈-alkylcarboxyl; aminocarbonyl; mono- or di-C₁-C₁₈alkylamino; C₁-C₁₀acylamino; or -COOH; and
M is an alkali metal ion.

2. Cosmetic formulation according to claim 1, wherein the particulate organic UV-filters (b) of formula (1) correspond to formula

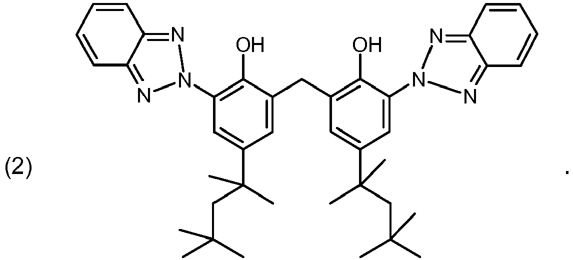

3. Cosmetic formulation according to claim 1, wherein the particulate organic UV-filter (b) of formula (3) corresponds to the formula

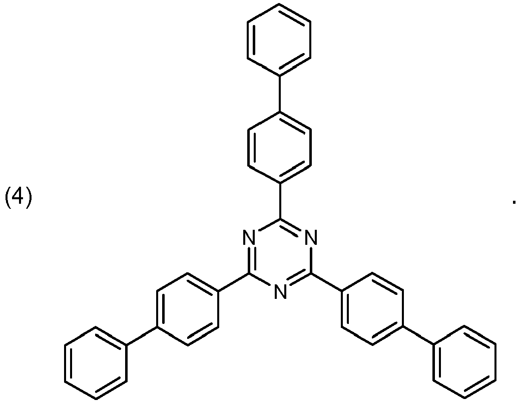

4. Cosmetic formulation according to claim 1, comprising
0.01 to 10 % b.w of particulates (a); and
0.1 to 40 % b.w of UV filters mixture (b).

5. Use of the formulation according to any of claims 1 to 4 for minimizing and/or masking the whitening effect and/or the particular color of a sunscreen composition.

6. Use of the formulation according to any of claims 1 to 4 for adjusting a natural skin-like color tone of the sunscreen formulation.

7. Use of the composition according to any of claims 1 to 4 for promoting an even skin tone.

8. Use of the composition according to any of claims 1 to 4 for minimising fine lines and wrinkles.

## Patentansprüche

1. Kosmetische Formulierung, umfassend
(a) Partikel mit einer Absorption in dem Bereich von 400 bis 800 nm, ausgewählt aus (a₁) Interferenzpigmenten auf der Grundlage von Glimmer, die mit Eisenoxid überzogen sind;
und
(b) UV-Filtern ausgewählt aus einem Gemisch von partikelförmigen organischen UV-Filtern ausgewählt aus den Verbindungen der Formel 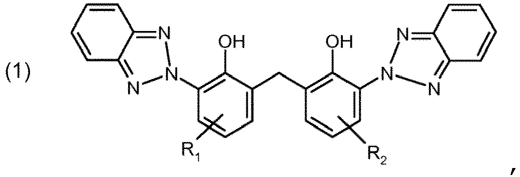 wobei
R₁ und R₂ unabhängig voneinander C₁-C₁₈Alkyl sind, das mit einem oder mehreren Resten ausgewählt aus C₁-C₄Alkyl, C₅-C₁₂Cycloalkyl und C₆-C₁₀Aryl substituiert sein können; und den Verbindungen der Formel 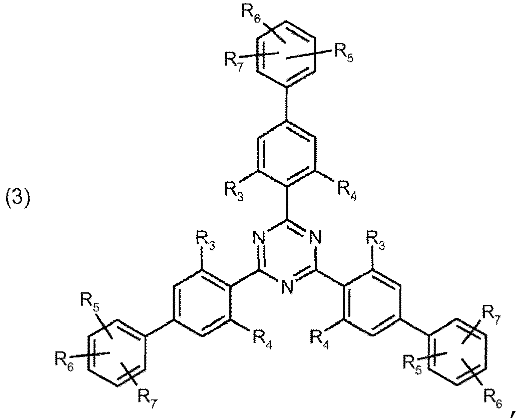 wobei
R₃ und R₄ unabhängig voneinander Wasserstoff; C₁-C₁₈Alkyl; oder C₆-C₁₂Aryl sind;
R₅, R₆ und R₇ unabhängig voneinander Wasserstoff; Hydroxy; Halogen; C₁-C₁₈Alkyl; C₁-C₁₈Alkoxy; C₆-C₁₂Aryl; Biphenylyl; C₆-C₁₂Aryloxy; C₁-C₁₈Alkylthio; Carboxy; -COOM; C₁-C₁₈Alkyl-carboxyl; Aminocarbonyl; Mono-oder Di-C₁-C₁₈alkylamino; C₁-C₁₀Acylamino; oder -COOH sind; und
M ein Alkalimetall-Ion ist.

2. Kosmetische Formulierung gemäß Anspruch 1, wobei die partikelförmigen organischen UV-Filter (b) der Formel (1) der Formel 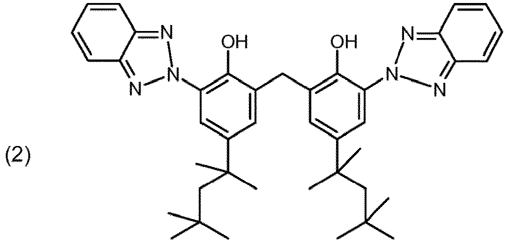 entsprechen.

3. Kosmetische Formulierung gemäß Anspruch 1, wobei die partikelförmigen organischen UV-Filter (b) der Formel (3) der Formel 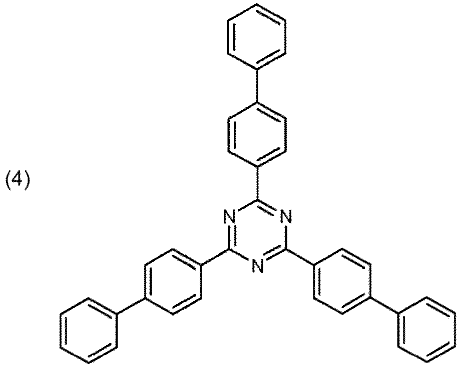 entsprechen.

4. Kosmetische Formulierung gemäß Anspruch 1, umfassend
0,01 bis 10 Gew.-% Partikel (a); und
0,1 bis 40 Gew.-% UV-Filtergemisch (b).

5. Verwendung der Formulierung gemäß einem der Ansprüche 1 bis 4 zum Minimieren und/oder Maskieren der weißmachenden Wirkung und/oder der besonderen Farbe einer Sonnenschutzzusammensetzung.

6. Verwendung der Formulierung gemäß einem der Ansprüche 1 bis 4 zum Einstellen eines natürlichen hautartigen Farbtons der Sonnenschutzformulierung.

7. Verwendung der Zusammensetzung gemäß einem der Ansprüche 1 bis 4 zum Fördern eines gleichmäßigen Hauttons.

8. Verwendung der Zusammensetzung gemäß einem der Ansprüche 1 bis 4 zum Minimieren feiner Linien und Falten.

## Revendications

1. Formulation cosmétique, comprenant :
(a) des matières particulaires ayant une absorption dans la plage allant de 400 à 800 nm, choisies parmi (a₁) des pigments d'interférence à base de mica et revêtus d'oxyde de fer ; et
(b) des filtres anti-UV choisis parmi un mélange de filtres anti-UV organiques particulaires choisis parmi les composés de formule (1) 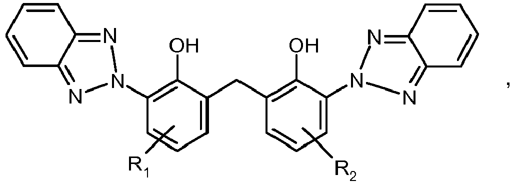 où
R₁ et R₂ sont indépendamment l'un de l'autre C₁-C₁₈-alkyle qui peut être substitué par un ou plusieurs radicaux choisis parmi C₁-C₄-alkyle, C₅-C₁₂-cycloalkyle et C₆-C₁₀-aryle ; et
les composés de formule (3) 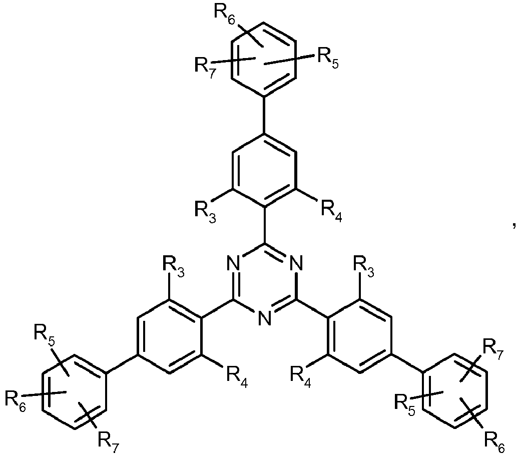 où
R₃ et R₄ sont indépendamment l'un de l'autre hydrogène ; C₁-C₁₈-alkyle ; ou C₆-C₁₂-aryle ;
R₅, R₆ et R₇ sont indépendamment les uns des autres hydrogène ; hydroxy ; halogène ; C₁-C₁₈-alkyle ; C₁-C₁₈-alcoxy ; C₆-C₁₂-aryle ; biphénylyle ; C₆-C₁₂-aryloxy ; C₁-C₁₈-alkylthio ; carboxy ; -COOM ; C₁-C₁₈-alkylcarboxyle ; aminocarbonyle ; mono- ou di-C₁-C₁₈-alkylamino ; C₁-C₁₀-acylamino ; ou -COOH ; et
M est un ion de métal alcalin.

2. Formulation cosmétique selon la revendication 1, dans laquelle les filtres anti-UV organiques particulaires (b) de formule (1) correspondent à la formule (2)

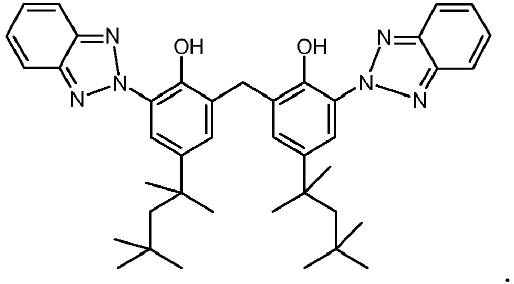

3. Formulation cosmétique selon la revendication 1, dans laquelle le filtre anti-UV organique particulaire (b) de formule (3) correspond à la formule (4)

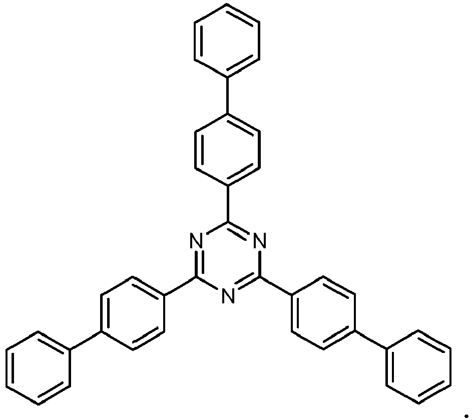

4. Formulation cosmétique selon la revendication 1, comprenant
de 0,01 à 10% en poids de matières particulaires (a) ; et
de 0,1 à 40% en poids de mélange de filtres anti-UV (b) .

5. Utilisation de la formulation selon l'une quelconque des revendications 1 à 4, afin de minimiser et/ou masquer l'effet de blanchiment et/ou la couleur particulière d'une composition d'écran solaire.

6. Utilisation de la formulation selon l'une quelconque des revendications 1 à 4, afin d'ajuster une teinte de type peau naturelle de la formulation d'écran solaire.

7. Utilisation de la composition selon l'une quelconque des revendications 1 à 4, afin de favoriser une teinte de peau uniforme.

8. Utilisation de la composition selon l'une quelconque des revendications 1 à 4, afin de minimiser les ridules et les rides.
